# EUROPEAN PATENT APPLICATION

(11) **EP 4 170 632 A1**
(43) Date of publication of application: **26.04.2023**
(21) Application number: 22800564.1
(22) Date of filing: 07.09.2022
(51) Int. Cl.: G09B 23/28, G09B 9/00, A61B 17/225, A61B 17/00

(54) **RENAL SURGERY TRAINING SYSTEM**

(30) Priority: 07.09.2021 KR 20210118902; 07.09.2021 KR 20210118910
(71) Applicant: ROEN Surgical, Inc., Daejeon 34051 (KR)
(72) Inventor: CHEON, Byung Sik, Yuseong-gu, Daejeon 34125 (KR); KIM, Chang Kyun, Yuseong-gu, Daejeon 34054 (KR); KWON, Dong Soo, Yuseong-gu, Daejeon 34051 (KR)
(74) Representative: Gill Jennings & Every LLP
(86) International application number: PCT/KR2022/013434
(87) International publication number: WO 2023/038424

(57) **Abstract**

A renal surgery training system includes: an internal calyx fluid simulation device including a supply hose configured to supply fluid, a simulator connected to the supply hose and through which the fluid supplied by the supply hose flows, a fluid receiver connected to the simulator such that the fluid flows and in which the fluid moved from the simulator is received, and an outlet hose configured to discharge the fluid stored in the fluid receiver, the internal calyx fluid simulation device configured to simulate a flow of fluid in a kidney; and a translation device disposed below the internal calyx fluid simulation device and configured to perform a translational movement to translate the internal calyx fluid simulation device, the renal surgery training system configured to provide training on removing an object swimming inside the kidney by a flow of fluid in the kidney by using training equipment.

## Description

### [Technical Field]

The following description relates to a renal surgery training system.

### [Background Art]

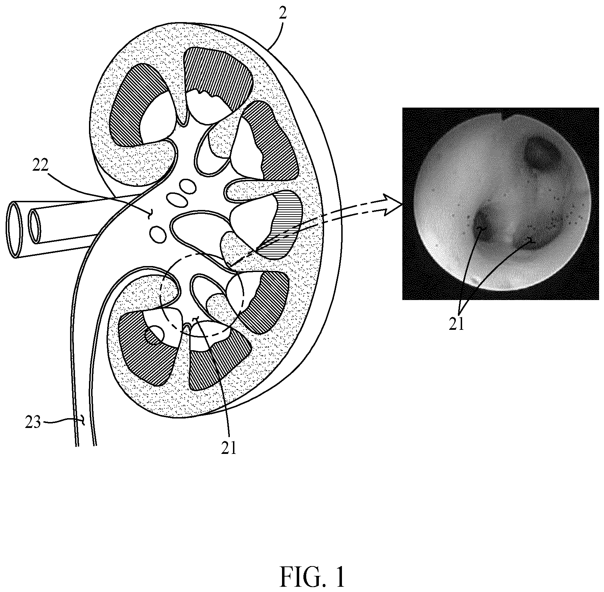

Flexible endoscopic instruments and devices have recently been developed and are being more widely used for treating stones or tumors in the kidneys and upper ureters. A flexible endoscope is designed to have a bendable distal end such that it moves up to a target minor calyx 21 through a major calyx 22 while moving along a ureter 23, as shown in FIG. 1. Correctly inserting the endoscope into a target surgical site requires a high level of skill which doctors may acquire through additional training. However, during an actual process of inserting the endoscope, a kidney continues moving irregularly as a patient breathes, a training system with such a condition reflected therein may thus be required. In addition, a stone continues swimming irregularly by the flow of fluid formed in the minor calyx 21 in the kidney, a training system that removes fast the stone from the kidney in such an environment may thus be required.

International Patent Publication No. WO 2015/095715 (published on June 25, 2015) discloses a simulator system for medical procedure training.

The above description is information the inventor(s) acquired during the course of conceiving the present disclosure, or already possessed at the time, and is not necessarily art publicly known before the present application was filed.

### [Disclosure of Invention]

### [Technical Goals]

An aspect of the present disclosure is to provide a renal surgery training system that includes a plurality of routes to simulate an internal structure of a kidney and provides a condition under which movements occur by respiration.

An aspect of the present disclosure is to provide a renal surgery training system that simulates the flow of fluid in a kidney and provides a condition under which movements occur by respiration.

### [Technical Solutions]

According to an embodiment, there is provided a renal surgery training system including: an internal calyx fluid simulation device including a supply hose configured to supply fluid, a simulator connected to the supply hose and through which the fluid supplied by the supply hose flows, a fluid receiver connected to the simulator such that the fluid flows and in which the fluid moved from the simulator is received, and an outlet hose configured to discharge the fluid stored in the fluid receiver, the internal calyx fluid simulation device configured to simulate a flow of fluid in a kidney; and a translation device disposed below the internal calyx fluid simulation device and configured to perform a translational movement to translate the internal calyx fluid simulation device, the renal surgery training system with which removing an object swimming in the kidney by the flow of the fluid in the kidney using training equipment is trained.

The simulator may include a first frame member forming an outermost shape of the simulator, an inlet channel formed by passing through the first frame member and communicating with the supply hose, a plurality of inlet holes communicating with an end of the inlet channel and configured to disperse the fluid moved from the inlet channel, a flow path communicating with the inlet holes and rising upward as approaching closer to the fluid receiver, and a cap covering a top of the flow path.

The flow path may be formed with a first flow path, a second flow path, and a third flow path respectively connected to a plurality of supply hoses, and one end of the first flow path, one end of the second flow path, and one end of the third flow path may be connected.

The inlet holes may be arranged on three side surfaces curved with respect to the inlet channel.

The object which is a training target may be disposed at a bottom of the flow path and configured to swim along a flow of the fluid from the inlet channel to the flow path through the inlet holes.

In a state in which the top of the flow path is open as the cap is opened, the object may be configured to be put into the flow path or taken out of the flow path.

The fluid receiver may include a second frame member connected such that the fluid is introduced from the simulator, an equipment insertion hole formed on one side of the second frame member and through which the training equipment passes, and a fluid storage space in which the fluid is stored.

The translation device may include a base, a bridge disposed on the base, a holder disposed on the bridge and configured to be movable in both directions along one axis with respect to the bridge, and a driver configured to drive the holder.

The bridge may be provided as a pair of bridges on both sides of the base, on which a sliding groove may be formed in a longitudinal direction of the bridge. The holder may include a slider configured to be movably inserted into the sliding groove, and a plate disposed on the slider and configured to move along with the slider.

The driver may include a body configured to provide rotational power, a wheel disposed on a side surface of the body and configured to be rotationally driven, a support having one side rotatably connected to the wheel and extending to the other side, and a connecting end to which the support is rotatably connected and connected to the holder.

The internal calyx fluid simulation device may further include a control valve disposed in the supply hose and configured to control a supply flow rate of the fluid.

The renal surgery training system may further include a calyceal structure simulation device including a frame into which the training equipment is inserted, a first route connected from one side of the frame up to a center of the frame and configured to simulate a ureter, a second route including a portion that is connected to the first route and has a cross-sectional area that increases as a distance from the first route increases, and configured to simulate a major calyx of the kidney, and a third route formed to branch into a plurality of routes from the second route and configured to simulate a minor calyx of the kidney, and the calyceal structure simulation device may be disposed on the translation device. Under a condition that the kidney moves by respiration, the renal surgery training system may provide training on inserting the training equipment along a route simulating an actual internal structure of the kidney.

The calyceal structure simulation device may further include a sensor disposed on one side of the third route and configured to sense whether the training equipment is reached.

The third route may be formed as a plurality of third routes at each of an upper end and a lower end of the frame, and the third routes may be alternately disposed at the upper end and the lower end along a lateral direction not to completely overlap each other when the calyceal structure simulation device is viewed from above.

The renal surgery training system may further include a control device configured to set a route which is an insertion target among a plurality of routes formed inside the calyceal structure simulation device, a determination device configured to determine whether the training equipment is inserted into the set route, and an output device configured to display whether the training equipment is inserted into the set route.

### [Effects]

A renal surgery training system according to an embodiment may simulate an internal structure of a kidney and provide a respiration condition to provide training on inserting training equipment into the kidney.

A renal surgery training system according to an embodiment may simulate the flow of fluid in a kidney, embody an object swimming by the fluid, and provide a respiration condition to provide training on inserting training equipment into the kidney and gripping a stone therein or aiming a laser correctly.

### [Brief Description of Drawings]

FIG. 1 is a cross-sectional view of a kidney and an enlarged view of a minor calyx.
FIG. 2 is a side view of a translation device and a calyceal structure simulation device according to an embodiment.
FIG. 3 is a block diagram illustrating a renal surgery training system according to an embodiment.
FIG. 4 is a cross-sectional view taken along the line A-A of FIG. 2.
FIG. 5 is a cross-sectional view taken along the line B-B of FIG. 4.
FIG. 6 is a perspective view of a translation device according to an embodiment.
FIG. 7 is a cross-sectional view taken along the line C-C of FIG. 6.
FIG. 8 is an image of a calyceal structure simulation device according to an embodiment.
FIG. 9 is a side view of a translation device and an internal calyx fluid simulation device according to an embodiment.
FIG. 10 is a cutaway cross-sectional view of a translation device and an internal calyx fluid simulation device according to an embodiment.
FIG. 11 is a cross-sectional view taken along the line D-D of FIG. 9.
FIG. 12 is a diagram illustrating an inlet channel and an inlet hole according to an embodiment.
FIG. 13 is an image of an internal calyx fluid simulation device according to an embodiment.
FIG. 14 is a diagram illustrating a configuration of a renal endoscopy simulator system according to an embodiment.
FIG. 15 is a perspective view of an artificial kidney module according to an embodiment.
FIG. 16 is a cross-sectional view taken along the line E-E of FIG. 15.
FIG. 17 is an exploded perspective view of an artificial kidney module according to an embodiment.
FIG. 18 is a diagram illustrating a configuration of a renal endoscopy simulator system according to an embodiment.

### [Best Mode for Carrying Out Invention]

Hereinafter, embodiments of the present disclosure will be described in detail with reference to the accompanying drawings. Regarding the reference numerals assigned to the components in the drawings, it should be noted that the same components are assigned with the same reference numerals, wherever possible, even though they are shown in different drawings. Further, in the following description of the embodiments, a detailed description of publicly known configurations or functions incorporated herein will be omitted when it is deemed that the detailed description obscures the subject matters of the embodiments.

In addition, the terms such as first, second, A, B, (a), and (b) may be used to describe constituent elements of the embodiments. These terms are used only for the purpose of discriminating one constituent element from another, and the nature, the sequence, or the order of the elements are not limited by the terms. When one element is described as being "connected," "coupled," or "attached" to another element, it should be understood that one element may be connected or attached directly to another element, and an intervening element may also be "connected," "coupled," or "attached" to the elements.

An element, which has the same common function as an element included in any one embodiment, will be described by using the same name in other embodiments. Unless otherwise stated, a description provided with reference to any one embodiment may be applied to other embodiments, and a more detailed and repeated description thereof will be omitted.

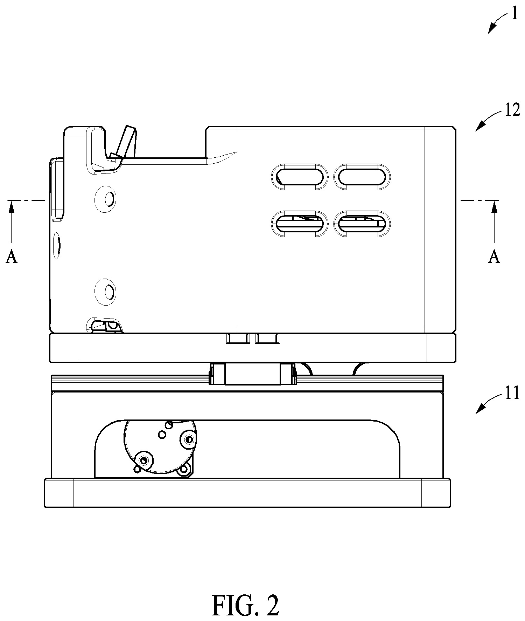

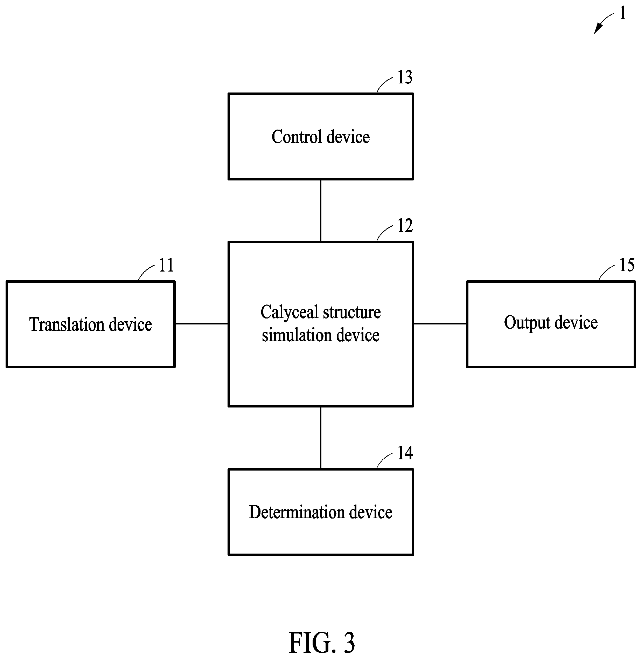

FIG. 2 is a side view of a translation device and a calyceal structure simulation device according to an embodiment, and FIG. 3 is a block diagram illustrating a renal surgery training system according to an embodiment.

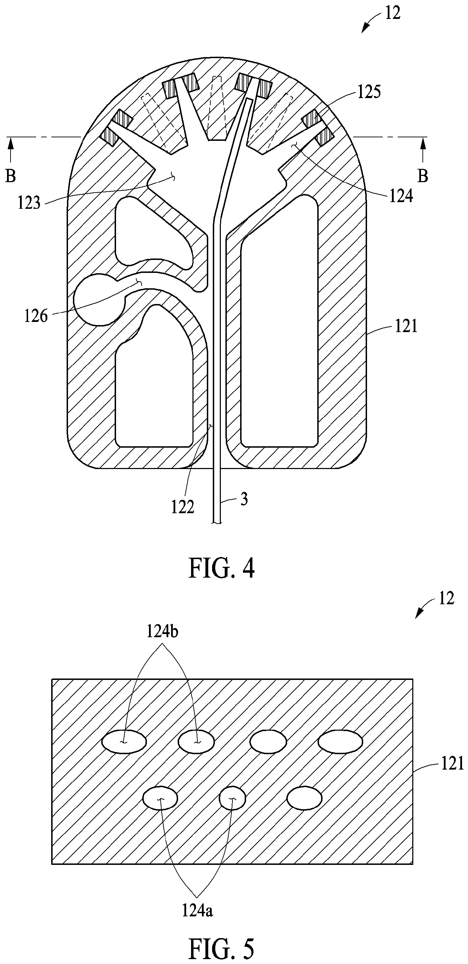

Referring to FIGS. 2 and 3, a renal surgery training system 1 may be used to provide training to insert training equipment (e.g., 3 of FIG. 4) along a route simulating an internal structure of an actual kidney 2 under a condition that the kidney 2 moves by respiration. For example, the renal surgery training system 1 may include a translation device 11, a calyceal structure simulation device 12, a control device 13, a determination device 14, and an output device 15.

The translation device 11 may be disposed below the calyceal structure simulation device 12 and may perform a translational movement to translate the calyceal structure simulation device 12. The translation device 11 may create an environment in which the kidney 2 moves by respiration in an actual body. Although a structure performing a translational movement in one direction is illustrated, examples are not limited thereto, and a typical structure that enables a translational or circular movement with respect to a plurality of axes may also be applicable.

The calyceal structure simulation device 12 may simulate the internal structure of the kidney 2 to provide training to access the inside of the kidney 2 using the training equipment 3. A plurality of branched routes may be formed inside the calyceal structure simulation device 12 to simulate a structure of a minor calyx 21 of the kidney 2.

The control device 13 may set a route which is an insertion target among the routes formed inside the calyceal structure simulation device 12.

The determination device 14 may determine whether the training equipment 3 is inserted in the set route. For example, the determination device 14 may determine whether the training equipment 3 is detected by a sensor (e.g., 125 of FIG. 4).

The output device 15 may indicate whether the training equipment 3 is inserted in the set route. For example, the output device 15 may be a means that enables visual checks with the naked eye, for example, a display, and may output a character string such as "reach" to inform in real time a user of whether a target route is reached when a route into which the training equipment 3 is inserted is the set route.

FIG. 4 is a cross-sectional view taken along the line A-A of FIG. 2, and FIG. 5 is a cross-sectional view taken along the line B-B of FIG. 4.

Referring to FIGS. 4 and 5, the calyceal structure simulation device 12 may include a frame 121, a first route 122, a second route 123, a third route 124, a fourth route 126, and a sensor 125.

The training equipment 3 may be inserted into the frame 121 to enter the inside thereof. For example, the frame 121 may be formed of a transparent or translucent material such that a route through which the training equipment 3 enters from the outside is identified with the naked eye.

The first route 122 may be connected from one side of the frame 121 up to a center of the frame 121 and may simulate a ureter 23.

The second route 123 may include a portion that is connected to the first route 122 and has a cross-sectional area that increases as a distance from the first route 122 increases and may simulate a major calyx 22 of the kidney 2.

The third route 124 may be formed to branch out into a plurality of routes from the second route 123 and may simulate the minor calyx 21 of a kidney. For example, the third route 124 may be provided as a plurality of third routes at each of an upper end and a lower end of the frame 121, and the plurality of third routes 124 may be alternately disposed at the upper end and the lower end along a lateral direction. Such a structure may prevent the plurality of third routes 124 from fully overlapping when the calyceal structure simulation device 12 is viewed from above and may simulate a similar structure to that of the actual kidney 2. In addition, it may complicate an operation of inserting the training equipment 3 from third routes 124a disposed at the upper end to third routes 124b disposed at the lower end, which may enable training with a similar difficulty to that of an actual procedure by using a pattern that is not simple.

The fourth route 126 may branch from the first route 122 or the second route 123 and may be curved to have a smaller radius of curvature than the third route 124. When the fourth route 126 is set as the target route, training may be performed to insert the training equipment 3 into the minor calyx 21 which is difficult to enter due to a small radius of curvature.

The sensor 125 may be disposed on one side of the third route 124 and may detect whether the training equipment 3 is reached. For example, the sensor 125 may be provided as a plurality of sensors, and the plurality of sensors may emit light in different colors depending on a position where each sensor 125 is disposed. By this characteristic, when the training equipment 3 is inserted into a route that is not the route set as the target route, it is possible to specify a route into which the training equipment 3 is inserted and correct the operation during the training.

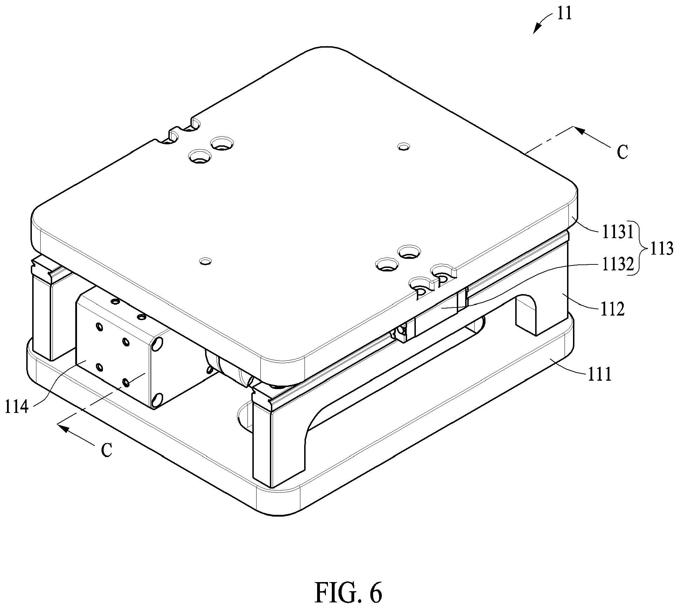

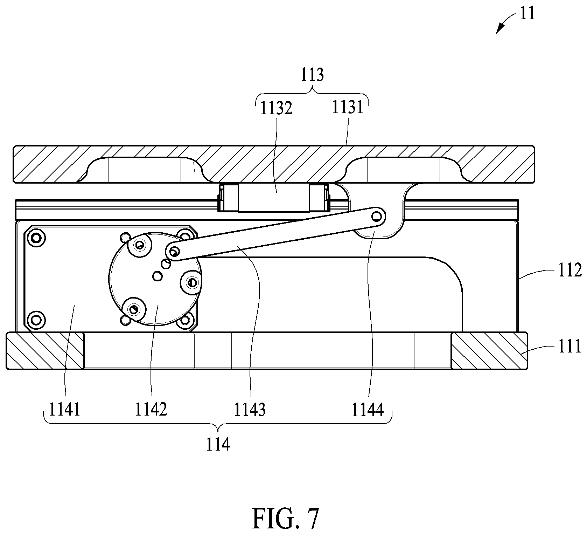

FIG. 6 is a perspective view of a translation device according to an embodiment, and FIG. 7 is a cross-sectional view taken along the line C-C of FIG. 6.

Referring to FIGS. 6 and 7, the translation device 11 may include a base 111, a bridge 112, a holder 113, and a driver 114.

The bridge 112 may be disposed on the base 111. For example, the bridge 112 may be provided as a pair of bridges on both sides of the base 111, on which a sliding groove may be formed in a longitudinal direction of the bridge 112.

The holder 113 may be disposed on the bridge 112 and may move in both directions along one axis with respect to the bridge 112. For example, the holder 113 may include a slider 1132 and a plate 1131.

The slider 1132 may be movably inserted into the sliding groove.

The plate 1131 may be disposed on the slider 1132 and may move along with the slider 1132.

The driver 114 may drive the holder 113. The driver 114 may adjust a movement range and a translation period of the holder 113. A control function of the driver 114 may simulate surgical situations with different respiration intensities and cycles, and adjust the level of difficulty of training by changing the degree of kidney movement. For example, the driver 114 may include a body 1141, a wheel 1142, a support 1143, and a connecting end 1144.

The body 1141 may provide rotational power.

The wheel 1142 may be disposed on a side surface of the body 1141 and may be rotationally driven.

The support 1143 may have one side rotatably connected to the wheel 1142 and extend long to the other side.

The connecting end 1144 may be connected to the holder 113, and to which the support 1143 may be rotatably connected.

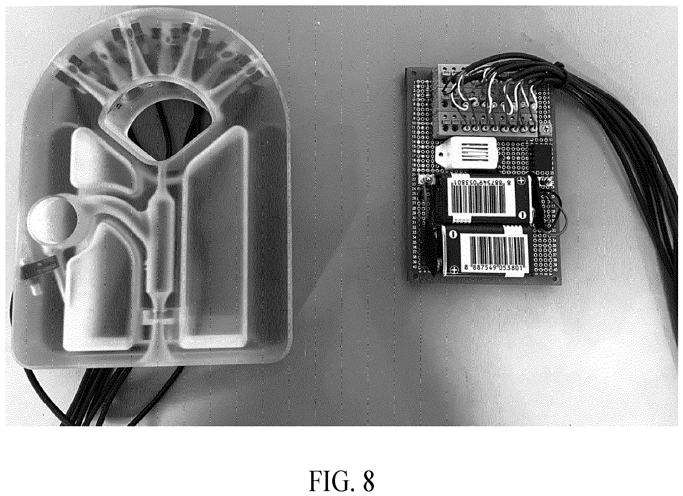

FIG. 8 is an image of a calyceal structure simulation device according to an embodiment.

Referring to FIG. 8, a plurality of routes formed through a translucent frame is shown, and a sensor disposed on each of the routes and a substrate connected to the sensor to emit light are shown. A user may be trained while directly observing the routes through the translucent frame, or may be trained on a sense of manipulation or handling the training equipment 3 by covering the top of the frame with an opaque material cover and determining whether the sensor emits light.

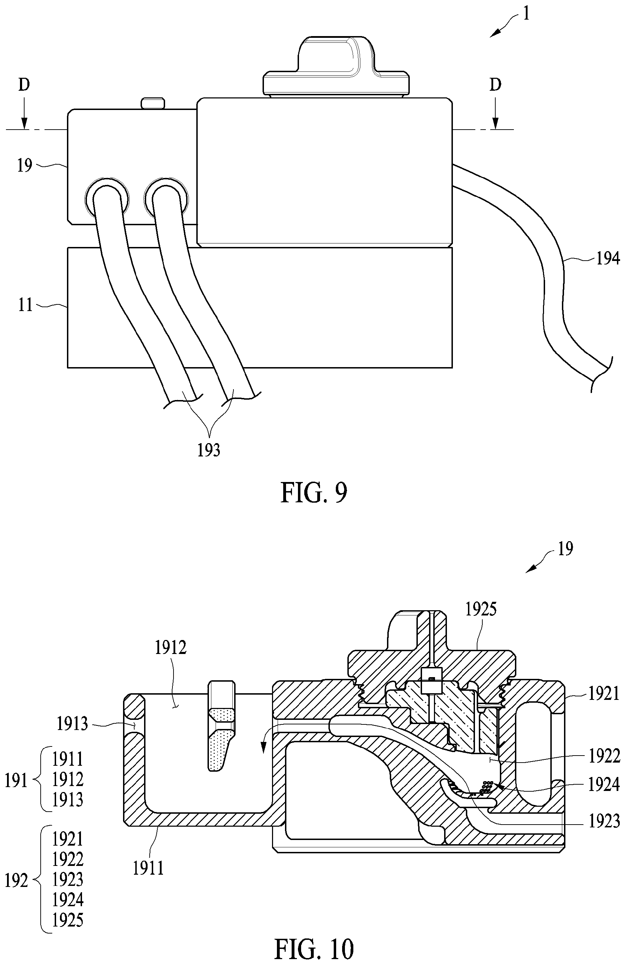

FIG. 9 is a side view of a translation device and an internal calyx fluid simulation device according to an embodiment.

Referring to FIG. 9, the renal surgery training system 1 may be used to provide training to allow an object such as a stone which is a surgical target to swim, grip the object with training equipment, or aim a laser correctly to irradiate the laser, in an environment simulating a flow of fluid flowing in an actual kidney 2, under a condition where the kidney 2 moves by respiration. For example, the renal surgery training system 1 may include the translation device 11 and an internal calyx fluid simulation device 19.

The translation device 11 may be disposed below the internal calyx fluid simulation device 19 and may translate the internal calyx fluid simulation device 19 through a translational movement thereof. The translation device 11 may create an environment in which the kidney 2 moves by respiration in an actual body. Although a structure that performs a translational movement in one direction is illustrated, examples are not limited thereto, and a typical structure that enables a translational or circular movement with respect to a plurality of axes may also be applicable.

The internal calyx fluid simulation device 19 may simulate an internal fluid flow in the kidney 2. For example, the internal calyx fluid simulation device 19 may form a flow that allows an object to swim as fluid is supplied from outside through a supply hose 194 and may discharge the fluid through an outlet hose 193.

The internal calyx fluid simulation device 19 may be aligned side by side with a calyceal structure simulation device above the translation device 11 or may be combined with the translation device 11 after the calyceal structure simulation device is separated from the translation device 11.

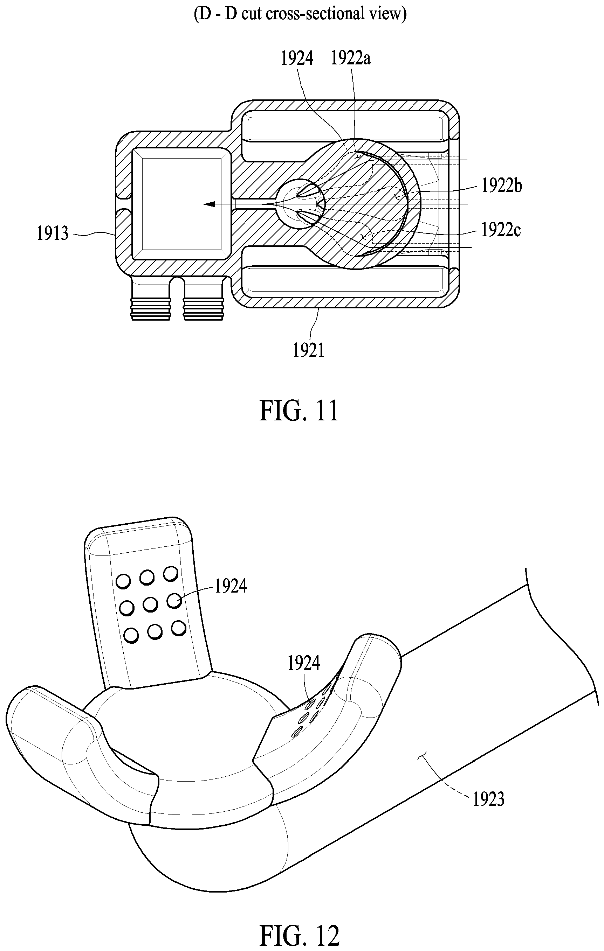

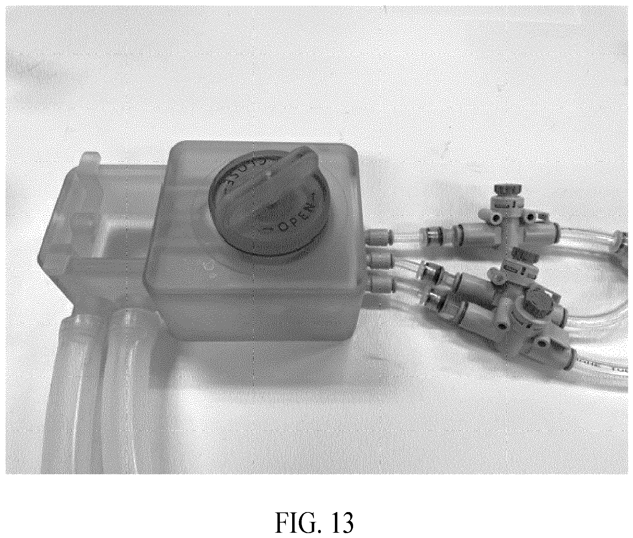

FIG. 10 is a cutaway cross-sectional view of a translation device and an internal calyx fluid simulation device according to an embodiment, FIG. 11 is a cross-sectional view taken along the line D-D of FIG. 9, FIG. 12 is a diagram illustrating an inlet channel and an inlet hole according to an embodiment, and FIG. 13 is an image of an internal calyx fluid simulation device according to an embodiment.

Referring to FIGS. 10 through 13, the internal calyx fluid simulation device 19 may include a fluid receiver 191, a simulator 192, an outlet hose 193, a supply hose 194, and a control valve (not shown).

The fluid receiver 191 may be connected to the simulator 192 such that fluid flows, and the fluid moved from the simulator 192 may be received therein. For example, the fluid receiver 191 may include a second frame member 1911, an equipment insertion hole 1913, and a fluid storage space 1912.

The second frame member 1911 may be connected such that the fluid is introduced from the simulator 192.

The equipment insertion hole 1913 may be formed on one side of the second frame member 1911 and allow training equipment to pass therethrough.

The fluid storage space 1912 may store fluid therein.

The simulator 192 may be connected to the supply hose 194, and the fluid supplied by the supply hose 194 may flow therethrough. For example, the simulator 192 may include a first frame member 1921, a flow path 1922, an inlet channel 1923, an inlet hole 1924, and a cap 1925.

The first frame member 1921 may form an outermost shape of the simulator 192.

The flow path 1922 may communicate with the inlet hole 1924 and may rise upward as it approaches closer to the fluid receiver 191. The flow path 1922 may rise as it approaches the fluid receiver 191, and the fluid flowing in from the inlet hole 1924 may be introduced into the fluid receiver 191 while flowing diagonally upward above the simulator 192 along the flow path 1922. For example, the flow path 1922 may be formed with a first flow path 1922a, a second flow path 1922b, and a third flow path 1922c respectively connected to a plurality of supply hoses 194, and one end of the first flow path 1922a, one end of the second flow path 1922b, and one end of the third flow path 1922c may be connected. An object, which is a training target, may be disposed at a bottom of the flow path 1922 and may swim along the flow of fluid from the inlet channel 1923 to the flow path 1922 through the inlet hole 1924.

The inlet channel 1923 may be formed by passing through the first frame member 1921 and may communicate with the supply hose 194.

The inlet hole 1924 may communicate with an end of the inlet channel 1923 and disperse the fluid moved from the inlet channel 1923. For example, the inlet hole 1924 may be provided as a plurality of inlet holes 1924, and the plurality of inlet holes 1924 may have a sufficiently large total cross-sectional area and may thus provide a flow velocity that is sufficiently large to allow the object to swim. The plurality of inlet holes 1924 may be disposed on three side surfaces curved with respect to the inlet channel 1923. Such a structure may allow the fluid flowing out of the inlet channel 1923 to be uniformly dispersed or sprayed through the inlet holes 1924 to prevent the object from moving only to one side and enable surgical training to be performed under various conditions.

The cap 1925 may cover the top of the flow path 1922. While the top of the flow path 1922 is opened by opening the cap 1925, the object may be put into or taken out from the flow path 1922.

The outlet hose 193 may discharge the fluid stored in the fluid receiver 191.

The supply hose 194 may supply fluid from the outside and may be connected to the simulator 192. For example, the supply hose 194 may be formed as many as the number of branched flow paths.

The control valve may be disposed in the supply hose 194 and control a supply fluid flow rate. With the flow rate under the control of the control valve, the intensity of turbulence may be varied, and the range and speed in and at which the object swims may be varied according to the intensity of turbulence. By controlling the degree to which the object swims, the level of difficulty of training may be adjusted.

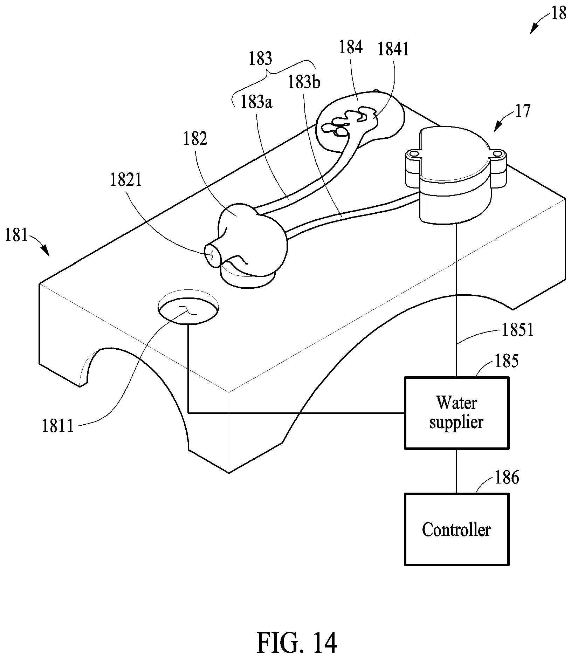

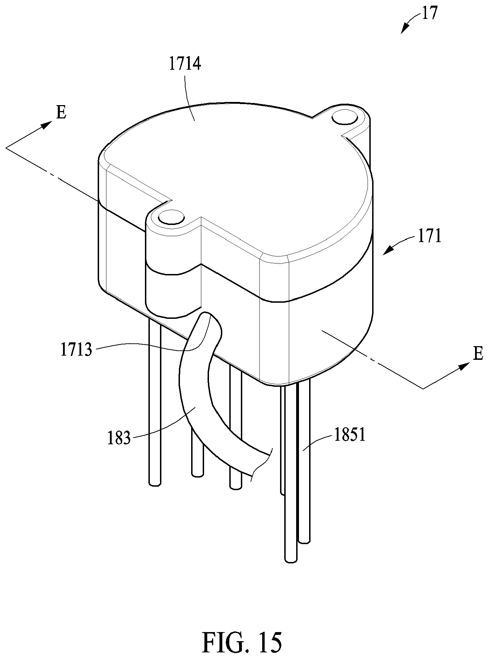

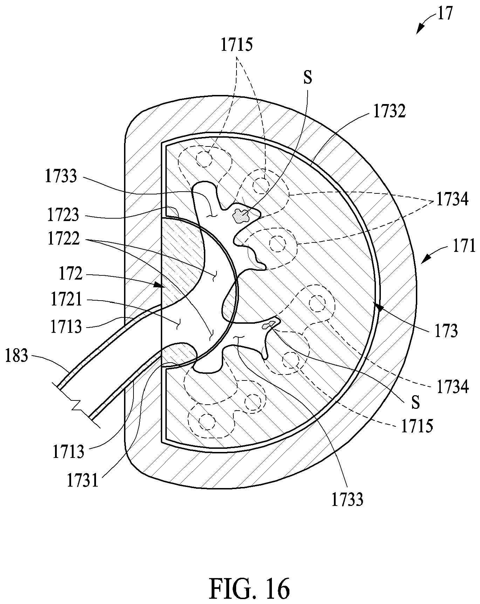

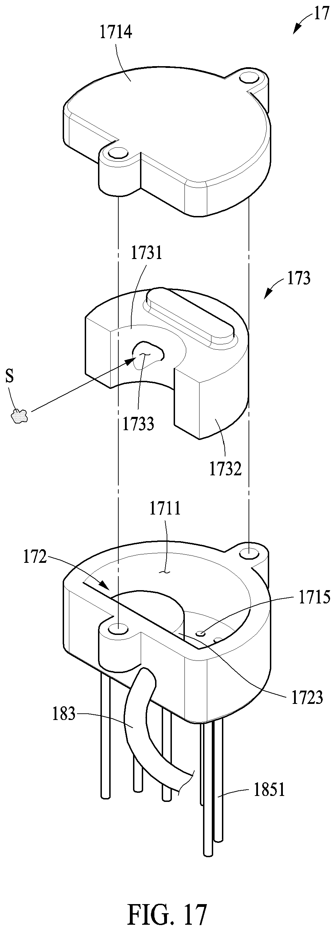

FIG. 14 is a diagram illustrating a configuration of a renal endoscopy simulator system according to an embodiment, FIG. 15 is a perspective view of an artificial kidney module according to an embodiment, FIG. 16 is a cross-sectional view taken along the line E-E of FIG. 15, and FIG. 17 is an exploded perspective view of an artificial kidney module according to an embodiment.

Referring to FIGS. 14 through 17, a renal surgery training system according to an embodiment may include a renal endoscopy simulator system 18 with which a kidney stone removal surgery using a ureteroscope is practiced.

According to an embodiment, the renal endoscopy simulator system 18 may include a base 181, an artificial bladder 182, an artificial ureter 183, a kidney model 184, a water supplier 185, a controller 186, and an artificial kidney module 17.

The base 181 may support the artificial bladder 182, the artificial ureter 183, the kidney model 184, and the artificial kidney module 17, on which the practice is conducted using the ureteroscope.

For example, the base 181 may include a drainage 1811 that drains water to a lower side of the artificial bladder 182 such that water supplied from the artificial kidney module 17 and flowed to the artificial bladder 182 is circulated to the water supplier 185. For example, the drainage 1811 may be a hole formed in the base 181 to communicate with the water supplier 185, or a housing that temporarily accommodates water, such as, for example, a water tank, and communicates with the water supplier 185.

The artificial bladder 182 may be a member in the form of a bladder having an internal space that simulates the shape of a human bladder. The artificial bladder 182 may have an opening 1821 that is connected to the artificial ureter 183 on one side and is exposed to the outside on the other side in an opposite direction such that the ureteroscope is introduced therethrough.

The water supplied through the artificial kidney module 17 may be connected to the artificial ureter 183 to be transferred to the artificial bladder 182, and the water to be discharged through the opening 1821 of the artificial bladder 182 may be discharged to the drainage 1811 disposed on the lower side by gravity.

For another example, a long tube-shaped member simulating the urethra of a human body may be additionally installed in addition to the opening 1821 of the artificial bladder 182.

For example, the artificial bladder 182 may be formed of a polymer material that is transmissive, such that a movement of an inserted tube of the ureteroscope passing through the inside is observable from the outside.

However, it should be noted that the artificial bladder 182 is not necessarily provided, and in such a case, the ureteroscope may be directly inserted through a discharge side of the artificial ureter 183.

The artificial ureter 183 may be a tube-shaped member that simulates a ureter of a human body. The artificial ureter 183 may be provided as a pair of artificial ureters 183 connected from the artificial bladder 182.

For example, of the pair of artificial ureters 183, one ureter 183 may be referred to herein as a first ureter 183a, and the other ureter 183 may be referred to herein as a second ureter 183b. In this example, a distinction between the first ureter 183a and the second ureter 183b is provided merely as an example of a distinction according to an actual structure as a pair of ureters respectively connected to a pair of kidneys, and there may be no specific directionality.

In addition, although the first ureter 183a is illustrated as being connected to the kidney model 184 and the second ureter 183b is illustrated as being connected to the artificial kidney module 17 in FIG. 14, this is provided merely as an example, and it should be noted that each artificial ureter (e.g., 183a and 183b) may be connected to any one of the components.

The kidney model 184 may be a member that simulates a kidney of a human body and may include an internal channel 1841 communicating with the artificial ureter 183 therein. The internal channel 1841 may be formed as a passage that branches into a plurality of channels from the artificial ureter 183 to allow the endoscope inserted through the artificial ureter 183 to access. The internal channel 1841 may have a shape that simulates an actual structure of a renal pelvis and renal calyces connected therefrom in a kidney.

For example, the artificial bladder 182, the kidney model 184, and the artificial ureter 183 may be formed of a polymer material that is transmissive, such that a movement of an inserted tube of the ureteroscope is observable from the outside. For example, the kidney model 184 may be formed of a transparent or translucent material, and a point at which the endoscope is inserted may thus be identified with the naked eye even from the outside. Thus, an unskilled user who is unfamiliar with manipulating or handling the endoscope may be first trained to insert the endoscope through the first ureter 183a and may thereby become accustomed to endoscopic surgery more readily. The artificial bladder 182, the kidney model 184, and the artificial ureter 183 may have the same colors as an actual human bladder, kidney, and ureter, respectively.

The water supplier 185 may supply water to the artificial kidney module 17 to simulate an actual movement of a stone flowing in a kidney. In addition, the artificial kidney module 17 may be cooled using the water supplied by the water supplier 185 without an additional cooler, and it is thus possible to create a laser-usable environment used to simulate the breaking of a stone.

For example, the water supplier 185 may embody a movement of a stone S accommodated in the artificial kidney module 17 by supplying water to the artificial kidney module 17 through a supply pump or the like. The water used to move the stone S may be discharged through the artificial ureter 183 into which the endoscope is inserted, without an additional discharge means. Such a structure may embody a movement of a stone S while continuously supplying water to the artificial kidney module 17 by the water supplier 185. For example, the water discharged through the drainage 1811 may be supplied back to the water supplier 185. This structure may embody a movement of a stone S through a circulation process of recovering the water discharged from the artificial bladder 182 back to the water supplier 185, without an additional supply of water. For example, the water supplier 185 may include a pump structure capable of accommodating water and supplying water to the outside at the same time.

For example, the water supplier 185 may include a plurality of supply lines 1851 connected to a plurality of calyx channels 1734 of a multi-channel portion 173 and configured to supply water to the calyx channels 1734, respectively. For example, the plurality of supply lines 1851 may each have a valve that opens and closes a corresponding supply line of the supply lines 1851. Such a structure may allow water to be supplied only to some of the calyx channels 1734 in which a stone S is positioned. The valve described above may be, for example, a valve capable of adjusting an opening degree. Such a valve may be used to individually adjust a degree of a movement of a stone S, for each of the calyx channels 1734.

However, unlike what is illustrated, the supply lines 1851 may be connected to the internal channel 1841 of the kidney model 184 to supply water to the internal channel 1841.

The controller 186 may control the water supplier 185 to adjust a flow rate and a flow velocity of water to be supplied to the artificial expansion module 17.

For example, the controller 186 may individually control a flow of water flowing in each of the supply lines 1851 and may thereby individually control a flow of water flowing into the calyx channels 1734. For example, the controller 186 may control an opening degree of each valve described above.

The artificial kidney module 17 may create an environment in which a stone is formed in an actual structure of a kidney, thereby enabling the practice of a stone removal surgery through an actual endoscopic operation. The artificial kidney module 17 may communicate with the artificial ureter 183 connected from the artificial bladder 182.

Although the artificial kidney module 17 is illustrated in FIGS. 14 through 17 as having a structure and a direction corresponding to a left kidney of a pair of kidneys of a human body, this is provided merely as an example, and a component having a structure and a direction corresponding to a kidney that is at a symmetrical position in an opposite direction may also be applicable.

The artificial kidney module 17 may include a case 171, a branch portion 172, the multi-channel portion 173, and a stone S.

The case 171 may be a housing-type member that accommodates therein the branch portion 172 and the multi-channel portion 173. For example, the case 171 may include an internal space 1711, a connecting portion 1713 through which the artificial ureter 183 is introduced into the internal space 1711, a cover 1714 that shields the internal space 1711 from the outside, and a plurality of supply ports 1715 into which a plurality of supply lines 1851 is introduced into the internal space 1711.

The branch portion 172 and the multi-channel portion 173 may be accommodated in the internal space 1711. The internal space 1711 may support the branch portion 172 and the multi-channel portion 173 with them being aligned with each other. For example, the internal space 1711 may have a semi-cylindrical shape corresponding to a shape in which the branch portion 172 and the multi-channel portion 173 are combined.

For example, as the shape of an inner wall of the internal space 1711 has a structure that fits the shape of an outer surface formed by the branch portion 172 and the multi-channel portion 173, the branch portion 172 and the multi-channel portion 173 may be fixedly supported by being fitted to the inner wall of the internal space 1711 and may be, at the same time, connected to be in close contact with each other.

As shown in FIGS. 16 and 17, a cross section of the internal space 1711 may have a circular shape including an arc. However, this is provided merely as an example, and the shape of the branch portion 172 and the multi-channel portion 173, in addition to the internal space 1711, is not limited thereto, but various shapes may also be implemented.

The connecting portion 1713 may be a port that passes through the inner wall of the case 171 from the outside to communicate with the internal space 1711, and to which an end of the artificial ureter 183 may be connected. For example, the connecting portion 1713 may be connected to an introduction tube 1721 of the branch portion 172 installed in the internal space 1711.

The cover 1714 may be detachably separated to shield the internal space 1711 from the outside. The cover 1714 may prevent water supplied to the internal space 1711 from flowing out to the outside of the case 171. A sealing member may be provided between the cover 1714 and the internal space 1711, and the water supplied to the internal space 1711 may thus be discharged through the artificial ureter 183 without being leaked to the outside.

For example, the cover 1714 may shield the internal space 1711 and simultaneously press the multi-channel portion 173 accommodated in the internal space 1711 to firmly fix it.

For example, the cover 1714 may be formed of a member of which a portion is transmissive such that the internal space 1711 shielded by the cover 1714 is observable from the outside.

To insert or remove a stone S into or from the multi-channel portion 173 as shown in FIG. 17, the cover 1714 may be separated from the internal space 1711, and then the multi-channel portion 173 may be withdrawn from the internal space 1711.

The plurality of supply ports 1715 may be installed in a portion of the internal space 1711 in which the multi-channel portion 173 is installed. The supply ports 1715 may be respectively connected to one sides of the plurality of calyx channels 1734 formed inside the multi-channel portion 173. The multi-channel portion 173 may have a columnar shape having an arc-shaped cross section, and accordingly the supply ports 1715 may also be formed in an arc-shaped region. That is, the supply ports 1715 may be radially arranged with respect to the branch portion 172. For example, the supply ports 1715 may be connected from under the internal space 1711 along the direction of gravity.

For example, when the multi-channel portion 173 is combined at a position at which it is accurately coupled to the branch portion 172 in the internal space 1711, the supply ports 1715 may be connected such that they are accurately engaged with the one sides of the calyx channels 1734, respectively.

That is, the multi-channel portion 173 may include a passage that allows the calyx channels 1734 and the supply ports 1715 to communicate with each other, and water supplied to the supply ports 1715 may be introduced up to the calyx channels 1734 through the passage.

By such a structure described above, the water supplied from the water supplier 185 to the artificial kidney module 17 may be individually supplied to each of the calyx channels 1734 such that an actual movement of a stone flowing in the renal calyces of an actual kidney is simulated.

The branch portion 172 may be installed in a portion of the internal space 1711 that is connected to the connecting portion 1713 and may form an internal path communicating from the artificial ureter 183 to the multi-channel portion 173. For example, the introduction tube 1721 of the branch portion 172 may communicate with the artificial ureter 183. The branch portion 172 may have a columnar shape having a semi-circular or sectoral cross section.

For example, the branch portion 172 may include the introduction tube 1721 that communicates with the artificial ureter 183 through the connecting portion 1713, a branch tube 1722 that branches from the introduction tube 1721 and communicates with the internal space 1711, and a coupling end 1723 connected to the multi-channel portion 173 and to which the branch tube 1722 is exposed.

The introduction tube 1721, which is a portion communicating with the artificial ureter 183, may have a tubular shape that simulates an actual portion of the renal pelvis of a kidney that is connected to a ureter.

The branch tube 1722 may have a tubular shape that is connected from the introduction tube 1721 and branches into a plurality of tubes toward the coupling end 1723 to be opened. For example, the branch tube 1722 may have a tubular shape that simulates an actual portion branching into two to three major calyces in the renal pelvis of a kidney.

The coupling end 1723 may be a portion in which an opening through which the branch tube 1722 is exposed is formed and may be coupled to a separating end 1731 of the multi-channel portion 173.

When the coupling end 1723 and the separating end 1731 are coupled to each other, the opening of the branch tube 1722 exposed at the coupling end 1723 may communicate with a calyx entrance 1733 exposed at the separating end 1731.

When the branch portion 172 and the multi-channel portion 173 are coupled, the coupling end 1723 and the separating end 1731 may have contact surfaces that fit each other in shape such that they are in close contact with each other at an accurate coupling position. For example, as shown in FIGS. 16 and 17, the coupling end 1723 may have a shape protruding along an arc, and accordingly the separating end 1731 may have a shape recessed inward along the same arc.

The multi-channel portion 173 may be installed in a portion of the internal space 1711 connected to the branch portion 172. The multi-channel portion 173 may form an internal path communicating from an internal path of the branch portion 172 and may be connected to the branch tube 1722 on one side and connected to the plurality of supply ports 1715 on the other side. The multi-channel portion 173 may be detachably installed in the internal space 1711.

For example, the multi-channel portion 173 may include the separating end 1731 coupled to the coupling end 1723 of the branch portion 172, a separating outer surface 1732 in close contact with the inner wall of the internal space 1711, the calyx entrance 1733 recessed inward from the separating end 1731, and the plurality of calyx channels 1734 connected from the calyx entrance 1733 and branching into a plurality of paths.

The separating end 1731 may be a portion in which the calyx entrance 1733 is exposed to the outside and may be coupled to the coupling end 1723 of the branch portion 172.

As described above, the separating end 1731 may have the recessed shape corresponding to the protruding shape of the coupling end 1723. For example, the separating end 1731 may form an arc-shaped contact surface recessed inward.

Such a structure of the coupling end 1723 and the separating end 1731 having such corresponding shapes may guide the multi-channel portion 173 and the branch portion 172 to have an accurate coupling position when they are coupled to each other, and may align the branch tube 1722 and the calyx entrance 1733 such that they are accurately engaged with each other. In addition, the coupling end 1723 and the separating end 1731 may be in surface contact with each other while in close contact with each other, except for a portion corresponding to the branch tube 1722 and the calyx entrance 1733, which may prevent water flowing inside from being leaked out.

However, the shape of the contact surface of each of the coupling end 1723 and the separating end 1731 may include other shapes having a structure allowing them to fit in shape to be in close contact.

The separating outer surface 1732 may be an outer portion that comes into close contact with the inner wall of the internal space 1711. The separating outer surface 1732 may have an outer cross-sectional shape that fits a cross-sectional shape of the inner wall of the internal space 1711 such that it comes into close contact with the inner wall of the internal space 1711.

As shown in FIGS. 16 and 17, when a portion of the inner wall of the internal space 1711 has a cylindrical shape, the separating outer surface 1732 may also have a cylindrical outer structure, and thus the separating outer surface 1732 may be arranged to be in close contact with the inner wall of the internal space 1711.

By the structure described above, the multi-channel portion 173 may be arranged to have an accurate coupling position in the internal space 1711, and thus the calyx entrance 1733 of the multi-channel portion 173 may be accurately engaged with the branch tube 1722 of the branch portion 172 to communicate therewith, and ends of the calyx channels 1734 of the multi-channel portion 173 may also be arranged to be accurately engaged with the supply ports 1715, respectively, to communicate therewith.

Thus, when installed in the internal space 1711, the multi-channel portion 173 may be stably and fixedly supported by both sides of the separating end 1731 and the separating outer surface 1732, which may prevent the multi-channel portion 173 from departing from the accurate coupling position in the internal space 1711.

In addition, by the outer structure of the cylindrical shape, a structure in which the calyx entrance 1733 connected to the branch tube 1722 on one side and to one or more calyx channels 1734 on the other side is exposed to the outside may be formed. The calyx entrance 1733 may include an opening portion exposed to the separating end 1731. The calyx entrance 1733 may have a tubular shape that simulates an actual portion branching into a plurality of minor calyces from a major calyx of an actual kidney and connected.

For example, the same number of calyx entrances 1733 as the number of branch tubes 1722 may be formed at points spaced apart from each other along the separating end 1731.

The calyx channels 1734 may be passages that branch to have one or more paths from the calyx entrance 1733 and are connected, and may be respectively connected to the supply ports 1715 at respective ends of the passages to receive water.

The calyx channels 1734 may have a tubular shape that simulates an actual structure of a plurality of minor calyces that branch from the major calyx of an actual kidney and are connected.

Thus, the shape of a passage branching from the introduction tube 1721 to the plurality of calyx channels 1734 through the branch tube 1722 and the calyx entrance 1733 to communicate may have a three-dimensional (3D) passage shape that branches from the renal pelvis of an actual kidney into the minor calyces through the major calyx to communicate.

In addition, even when the artificial kidney module 17 has a 3D structure in which heights of the calyx channels 1734 are different, it is possible to insert a stone S into each of the calyx channels 1734 through the calyx entrance 1733 exposed to the outside, in a state in which the multi-channel portion 173 is separated. Thus, compared to a general artificial kidney module that disposes a stone S on the same two-dimensional (2D) plane, it is possible to provide more realistic training.

When the multi-channel portion 173 and the branch portion 172 are coupled at the accurate coupling position in the internal space 1711 as shown in FIG. 16, the calyx channels 1734 may be respectively engaged with the supply ports 1715 to be coupled, and it is thus possible to supply water individually to the calyx channels 1734.

For example, each calyx channel 1734 may include a structure that slopes downward along the direction of gravity as it goes toward a supply port 715 from the calyx entrance 1733.

By such a structure described above, when a stone S is arranged in a portion corresponding to a calyx channel 1734 or the calyx entrance 1733, the stone S may receive a force to move toward an end of the calyx channel 1734 by gravity and may simultaneously receive a force in a direction receding from the end of the calyx channel 1734 by water supplied through a corresponding supply port 1715 and flowing backward. Thus, the stone S may have an irregularly turbulent movement while floating at the calyx entrance 1733 or the calyx channel 1734, and it is thus possible to form an actual movement of a stone in an actual kidney.

For example, portions in which the supply ports 1715 are installed in the internal space 1711 may be installed at points radially spaced from each other with respect to an arc shape of the separating end 1731.

That is, the supply ports 1715 may have different radial angles each formed based on the arc shape.

Accordingly, the calyx channels 1734 of the multi-channel portion 173 may also be radially distributed based on the arc shape within a structurally possible orientation range.

For example, the supply ports 1715 may be respectively connected to the calyx channels 1734 by protruding in a direction vertical to a radial plane with respect to the arc shape of the separating end 1731.

In this case, the vertical direction may be an opposite direction to the direction of gravity, through which a flow of water supplied to a calyx channel 1734 may form turbulence that effectively allows a stone S to float.

For example, a channel structure that branches and extends into the calyx entrance 1733 and the calyx channels 1734 of the multi-channel portion 173 through the introduction tube 1721 and the branch tube 1722 of the branch portion 172 may form a stereoscopic passage structure that is bent and branches along a 3D direction, which is similar to an internal structure of an actual kidney.

For example, the branch portion 172 and the multi-channel portion 173 may have the same color as an actual kidney. For example, the branch portion 172 and the multi-channel portion 173 may be formed of a polymer material that is transmissive, such that a movement of an inserted tube of a ureteroscope passing through the inside is observable from the outside. However, unlike this, they may also be formed of an opaque or translucent red material to provide a similar environment to an actual environment in which an endoscope is inserted.

A stone S described herein may be a member corresponding to a stone produced in an actual kidney and may be inserted into the calyx entrance 1733 or the calyx channels 1734 of the multi-channel portion 173.

For example, the stone S may have a similar shape and size to those of an actual stone in an actual kidney and may have a similar mass and stiffness to those of the actual stone. For example, the stone S may be a kidney stone removed from an actual kidney.

As shown in FIG. 17, by separating the multi-channel portion 173 from the internal space 1711 while the cover 1714 shielding the internal space 1711 is separated, it is possible to readily insert the stone S in a state in which the calyx entrance 1733 formed at the separating end 1731 of the multi-channel portion 173 is exposed to the outside.

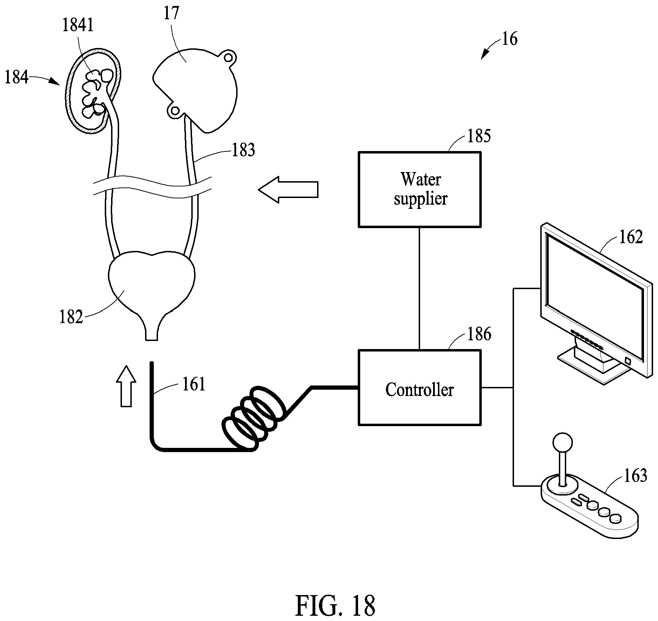

FIG. 18 is a diagram illustrating a configuration of a renal endoscopy simulator system according to an embodiment.

Referring to FIG. 18, a renal endoscopy simulator system 16 according to an embodiment may include an artificial bladder 182, an artificial ureter 183, a kidney model 184, a water supplier 185, an artificial kidney module 17, a controller 186, a ureteroscope 161, a display 162, and a manipulator 163.

The ureteroscope 161 may enter an opening of the artificial bladder 182 to enter the inside of the artificial kidney module 17 through the artificial ureter 183.

The display 162 may display in real time an image captured by a camera installed at a distal end of the ureteroscope 161.

The manipulator163 may control the driving of the ureteroscope 161 inserted into the artificial kidney module 17 through the artificial ureter 183.

Through the renal endoscopy simulator system 16 according to an embodiment, a user may manipulate the ureteroscope 161 with the manipulator 163 to introduce the ureteroscope 161 into the inside of the artificial kidney module 17 through the artificial ureter 183, and may then practice a procedure for removing a stone S disposed in the artificial kidney module 17 and observe such a procedure through the display 162 at the same time.

Through the renal endoscopy simulator system 16 according to an embodiment, it is possible to control the driving of the water supplier 185 through the controller 186 such that a stone S installed inside the multi-channel portion 173 has a turbulent movement in the calyx entrance 1733 or a calyx channel 1734 to simulate an actual movement formed by a stone in an actual kidney, using an image captured by the camera of the ureteroscope 161.

In addition, the controller 186 may adjust a flow rate and a flow velocity of water supplied through a supply line 1851 connected to a calyx channel 1734 in which a stone S is disposed through the water supplier 185, and the user may thus adjust in real time a desirable water supply flow rate and velocity while checking, through the display 162, the image of the stone S captured by the ureteroscope 161.

A number of embodiments have been described above. Nevertheless, it should be understood that various modifications may be made to these embodiments. For example, suitable results may be achieved if the described techniques are performed in a different order, and/or if components in a described system, architecture, device, or circuit are combined in a different manner and/or replaced or supplemented by other components or their equivalents.

## Claims

1. A renal surgery training system, comprising:
an internal calyx fluid simulation device comprising a supply hose configured to supply fluid; a simulator connected to the supply hose, through which the fluid supplied by the supply hose flows; a fluid receiver connected to the simulator such that the fluid flows, in which the fluid moved from the simulator is received; and an outlet hose configured to discharge the fluid stored in the fluid receiver, the internal calyx fluid simulation device configured to simulate a flow of fluid in a kidney; and
a translation device disposed below the internal calyx fluid simulation device and configured to perform a translational movement to translate the internal calyx fluid simulation device,
the renal surgery training system with which removing an object swimming in the kidney by the flow of the fluid in the kidney using training equipment is trained.

2. The renal surgery training system of claim 1, wherein the simulator comprises:
a first frame member forming an outermost shape of the simulator;
an inlet channel formed by passing through the first frame member and communicating with the supply hose;
a plurality of inlet holes communicating with an end of the inlet channel and configured to disperse the fluid moved from the inlet channel;
a flow path communicating with the inlet holes and rising upward as approaching closer to the fluid receiver; and
a cap covering a top of the flow path.

3. The renal surgery training system of claim 2, wherein the flow path is formed with a first flow path, a second flow path, and a third flow path respectively connected to a plurality of supply hoses, wherein one end of the first flow path, one end of the second flow path, and one end of the third flow path are connected.

4. The renal surgery training system of claim 2, wherein the inlet holes are arranged on three side surfaces curved with respect to the inlet channel.

5. The renal surgery training system of claim 2, wherein the object which is a training target is disposed at a bottom of the flow path and configured to swim along a flow of the fluid from the inlet channel to the flow path through the inlet holes.

6. The renal surgery training system of claim 2, wherein, in a state in which the top of the flow path is open as the cap is opened, the object is configured to be put into the flow path or taken out of the flow path.

7. The renal surgery training system of claim 1, wherein the fluid receiver comprises:
a second frame member connected such that the fluid is introduced from the simulator;
an equipment insertion hole formed on one side of the second frame member, through which the training equipment passes; and
a fluid storage space in which the fluid is stored.

8. The renal surgery training system of claim 1, wherein the translation device comprises:
a base;
a bridge disposed on the base;
a holder disposed on the bridge and configured to be movable in both directions along one axis with respect to the bridge; and
a driver configured to drive the holder.

9. The renal surgery training system of claim 8, wherein the bridge is provided as a pair of bridges on both sides of the base, on which a sliding groove is formed in a longitudinal direction of the bridge, and
the holder comprises:
a slider configured to be movably inserted into the sliding groove; and
a plate disposed on the slider and configured to move along with the slider.

10. The renal surgery training system of claim 8, wherein the driver comprises:
a body configured to provide rotational power;
a wheel disposed on a side surface of the body and configured to be rotationally driven;
a support having one side rotatably connected to the wheel and extending to the other side; and
a connecting end to which the support is rotatably connected, connected to the holder.

11. The renal surgery training system of claim 1, wherein the internal calyx fluid simulation device further comprises:
a control valve disposed in the supply hose and configured to control a supply flow rate of the fluid.

12. The renal surgery training system of claim 1, further comprising:
a calyceal structure simulation device comprising a frame into which the training equipment is inserted; a first route connected from one side of the frame up to a center of the frame and configured to simulate a ureter; a second route comprising a portion that is connected to the first route and has a cross-sectional area that increases as a distance from the first route increases, and configured to simulate a major calyx of the kidney; and a third route formed to branch into a plurality of routes from the second route and configured to simulate a minor calyx of the kidney, the calyceal structure simulation device disposed on the translation device,
wherein, under a condition that the kidney moves by respiration, the renal surgery training system is configured to provide training on inserting the training equipment along a route simulating an actual internal structure of the kidney.

13. The renal surgery training system of claim 12, wherein the calyceal structure simulation device further comprises:
a sensor disposed on one side of the third route and configured to sense whether the training equipment is reached.

14. The renal surgery training system of claim 12, wherein the third route is formed as a plurality of third routes at each of an upper end and a lower end of the frame, the third routes alternately disposed at the upper end and the lower end along a lateral direction not to completely overlap each other when the calyceal structure simulation device is viewed from above.

15. The renal surgery training system of claim 12, further comprising:
a control device configured to set a route which is an insertion target among a plurality of routes formed inside the calyceal structure simulation device;
a determination device configured to determine whether the training equipment is inserted into the set route; and
an output device configured to display whether the training equipment is inserted into the set route.
